# EUROPÄISCHE PATENTSCHRIFT

(11) **EP 1 265 542 B1**
(45) Veröffentlichungstag und Bekanntmachung des Hinweises auf die Patenterteilung: **31.03.2004**
(21) Anmeldenummer: 01905692.8
(22) Anmeldetag: 26.01.2001
(51) Int. Cl.: A61B 17/86, A61F 2/08, A61L 31/00

(54) **INTERFERENZSCHRAUBE AUS KNOCHENMATERIAL**
INTERFERENCE SCREW MADE OF BONE MATERIAL
VIS D'INTERFERENCE EN MATIERE OSSEUSE

(30) Priorität: 24.03.2000 DE 10014617
(43) Veröffentlichungstag der Anmeldung: 18.12.2002
(73) Patentinhaber: Tutogen Medical GmbH, 91077 Neunkirchen am Brand (DE)
(72) Erfinder: LÖWEL, Matthias, 90408 Nürnberg (DE); LANDIS, Klaus, 90562 Heroldsberg (DE); KOSCHATZKY, Karl, 91058 Erlangen (DE)
(74) Vertreter: Manitz, Finsterwald & Partner GbR
(86) Internationale Anmeldenummer: PCT/EP2001/000862
(87) Internationale Veröffentlichungsnummer: WO 2001/072233

(56) Entgegenhaltungen:
- EP-A- 0 556 571
- DE-A- 2 906 650
- US-A- 4 950 270
- US-A- 5 439 684
- US-A- 5 470 334
- US-A- 5 868 749
- US-A- 5 951 560
- US-A- 6 045 554

## Beschreibung

Die vorliegende Erfindung betrifft ein Implantat zur Fixation von Knochen insbesondere von Knochenblöcken mit Sehnenanteil, besonders bei der VKB Plastik (Vorderen Kreuzband Plastik).

Die VKB ist eine Behandlungsmethode für Patienten mit teilweiser oder vollständiger Ruptur des vorderen Kreuzbandes, bei der üblicherweise ein autologer bzw. allogener Patellaknochenblock-Patellasehne-Tibiaknochenblock (BTB, Bone-Tendon-Bone) in das Kniegelenk als Kreuzbandersatz implantiert wird.

Die Fixation der implantierten Knochenblöcke im Bohrkanal des Femurs und der Tibia erfolgt mit entsprechend dimensionierten Interferenzschrauben. Diese bestehen beispielsweise aus Metall (z.B. Titan) oder aus Kunststoffen (z.B. Polylactid).

Bei metallischen Implantaten besteht jedoch die Gefahr der Verletzung bzw. Zerstörung der implantierten Knochenblöcke bzw. des Sehnenanteils während der Implantation. Außerdem verbleiben diese Implantate lebenslänglich im Implantatlager und führen bei diagnostischen Untersuchung wie z.B. Röntgen zu Artefaktbildungen in der Bildgebung. Somit ist eine Diagnose über den Einheilungserfolg nach der Operation nur bedingt möglich.

Implantate aus Kunststoffen werden zwar über Monate hinweg abgebaut, die dabei entstehenden Mono- bzw. Oligomere können aber zu einer Schädigung des Implantates bzw. des Implantatlagers (Knochen) mit entsprechenden Komplikationen, wie z.B. Lyse des Knochens führen.

Eine Interferenzschraube nach dem Oberbegriff des Anspruchs 1 ist aus der US-A-4,950,270 bekannt.

In der US-A-5,868,749 sind verschiedene Schrauben aus Knochenmaterial offenbart. Der Kopf dieser Schrauben kann als Außenvierkant oder Außensechskant ausgebildet sein und größere Außenabmessungen als der Schraubenkörper aufweisen.

Es ist die Aufgabe der vorliegenden Erfindung, ein Implantat zur Fixation von Knochenelementen bzw. -blöcken zu schaffen, das die o.g. Nachteile beseitigt und das für eine zuverlässige Fixierung im bzw. am Patientenknochen sorgt, und das beim Einschrauben nicht beschädigt wird.

Diese Aufgabe wird gelöst durch eine Interferenzschraube mit den Merkmalen des Anspruchs 1.

Ein besonderer Vorteil der Interferenzschraube gemäß der Erfindung ist durch das verwendete Material gegeben, das aufgrund seines biologischen Ursprungs keinen Fremdkörper darstellt. Dadurch trägt das aus Knochenmaterial hergestellte Implantat zur Fixation und Fusion zwischen Implantat und Implantatlager bei, indem es sich während der Einheilung in körpereigenes Gewebe umwandelt.

Die erfindungsgemäße Interferenzschraube wird über einen längeren Zeitraum abgebaut und durch körpereigenes Gewebe ersetzt ohne eine Lyse des Implantates bzw. Implantatlagers durch Abbauprodukte zu verursachen. Die Interferenzschraube weist eine zentrale Durchgangsbohrung auf, so daß sie mit Hilfe eines entsprechenden Führungswerkzeugs definiert in das Implantatiager eingebracht werden kann. Das Außengewinde gewährleistet hierbei eine zuverlässige Verankerung des Implantats im Implantatlager ohne das Implantat mechanisch zu beschädigen.

Eine oder mehrere Spannuten, die an dem Schraubenkörper vorgesehen sind, gewährleisten das Ausbringen von autologem Knochen- bzw. Gewebematerial aus dem Implantatlager während des Eindrehens der Interferenzschraube.

Durch die Spannuten wird beim Einschrauben der Interferenzschraube gewährleistet, daß eventuell anfallende Knochen- bzw. Gewebefragmente aus dem Implantatlager bzw. Bohrkanal heraustransportiert werden, und so eine Beschädigung der Interferenzschraube bzw. des Implantatlagers verhindert wird.}

Die Fixation kann durch ein geeignetes Applikationsinstrument erfolgen, das auf einen am Schraubenkopf vorgesehenen Außenvierkant bzw. Außensechskant aufgebracht wird.

Die erfindungsgemäße Interferenzschraube weist am Schraubenkopf einen Außenvierkant, bevorzugt einen Außensechskant auf. Dadurch wird im Gegensatz zum Innenvierkant bzw. Innensechskant eine bessere und gleichmäßigere Kraftübertragung vom Setzwerkzeug auf die Interferenzschraube gewährleistet und somit ein Abdrehen des Schraubenkopfes vom Schraubenkörper während Implantation verhindert.

Durch die verschieden großen Abmessungen der Interferenzschraube ist neben einer Anwendung im femoralen und tibialen Tunnel im Rahmen einer VKB-Plastik auch eine Anwendung bei anderen chirurgischen Maßnahmen wie z.B. Fixation von Knochenfragmenten nach Frakturen möglich.

Vorteilhafte Ausführungsformen sind in der Beschreibung, der Zeichnung und den Unteransprüchen beschrieben.

Nach einer bevorzugten Ausführungsform der Erfindung besteht das Material der Interferenzschraube aus konserviertem und sterilen Knochenmaterial humanen oder tierischen Ursprungs.

Das Knochenmaterial kann aus spongiösem, kortikalem oder kompakten Knochen bzw. aus daraus resultierenden Verbunden bestehen.

Bei einer besonders vorteilhaften Ausgestaltung der erfindungsgemäßen Interferenzschraube weist der Schraubenkörper in Längsrichtung die Form eines Rotationsellipsoids auf. Eine solche Gestaltung erhöht die Stabilität der Fixation zwischen Implantat und Implancatlager.

Gemäß einer bevorzugten Ausführungsform der vorliegenden Erfindung ist die Bohrung, vorzugsweise eine Durchgangsbohrung derart gestaltet, daß unter Zuhilfenahme eines Führungsinstrumentariums eine Implantation ins definierte Implantatlager möglich ist. Ggf. kann durch bildgebende Verfahren die Fixation während der Operation kontrolliert werden.

Die Interferenzschraube ist in ihrer Größe an den Bohrkanal im femoralen bzw. tibialen Tunnel angepaßt wobei ihr Durchmesser im allgemeinen ca. 2 mm kleiner als der entsprechende Bohrkanal ist.

Die Applikation des BTB-Implanats und die anschließende Fixierung mit den entsprechenden Interferenzschrauben erfolgt nach Setzung der Bohrkanäle in den Femur und Tibia meist durch athroskopische Operationstechnik.

Als Material wird für die Interferenzschraube gemäß der vorliegenden Erfindung ein geeignetes allogenes oder xenogenes Knochenmaterial derart prozessiert, daß es konserviert, lagerfähig sowie steril ist und bestimmungsgemäß eingesetzt werden kann. Die Konservierung des Knochenmaterials kann beispielsweise mittels Gefriertrocknung erfolgen. Vorzugsweise wird aber das Knochenmaterial durch Lösungsmitteldehydratisierung von kollagenem Knochenmaterial mittels eines organischen mit Wasser mischbaren Lösungsmittels, z.B. Methanol, Ethanol, Propanol, Isopropanol, Aceton, Methyl-Ethylketon oder Gemischen dieser Lösungsmittel, erzeugt. Die Konservierung und Sterilisation des Knochenmaterials nach diesem Verfahren ist auch Gegenstand des Patents DE 29 06 650, dessen Inhalt durch Bezugnahme in die Offenbarung der vorliegenden Anmeldung aufgenommen wird.

Dieses Verfahren dient der Herstellung von Transplantatkonserven und ermöglicht eine Dehydratisierung und Freilegung bis in den Feinbau der Fibrille des kollagenen Knochenmaterials, so daß das prozessierte Knochenmaterial im histologischen Bild eine dem natürlichen Knochen sehr ähnliche Struktur aufweist und somit die gewünschten Eigenschaften des kollagenen Knochenmaterials erhalten bleiben. Dieses Verfahren der Lösungsmitteldehydratisierung hat außerdem den Vorteil, daß im Vergleich zur Gefriertrocknung ein wesentlich geringerer apparativer Aufwand erforderlich ist.

Ferner kann das Knochenmaterial auch durch Lösungsmitteldehydratisierung von kollagenem Knochenmaterial mit anschließender terminaler Sterilisation, insbesondere durch Bestrahlung mit Gamma- bzw. Elektronenstrahlen, aber auch durch Ethylenoxid oder thermische Verfahren hergestellt werden.

Alternativ kann das Knochenmaterial durch aseptische Prozessierung von kollagenem Knochenmaterial ohne terminale Sterilisation erzeugt werden.

Nachfolgend wird die vorliegende Erfindung rein exemplarisch anhand eines Ausführungsbeispiels und unter Bezugnahme auf die beigefügten Zeichnungen beschrieben. Es zeigen:
- Fig. 1: eine teilweise geschnittene Seitenansicht einer Interferenzschraube gemäß der vorliegenden Erfindung;
- Fig. 2: eine um 90° gedrehte Seitenansicht der Interferenzschraube von Fig. 1; und
- Fig. 3: eine Draufsicht auf den Schraubenkopf der Interferenzschraube von Fig. 1 und 2.

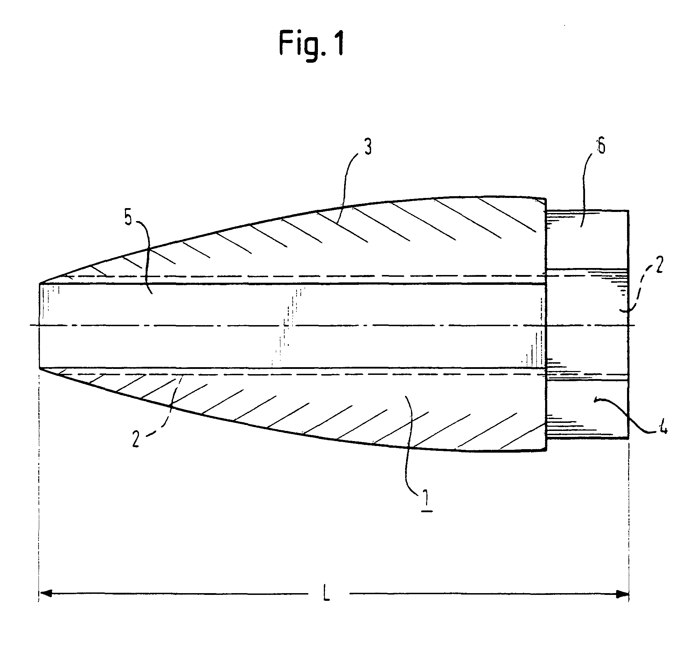

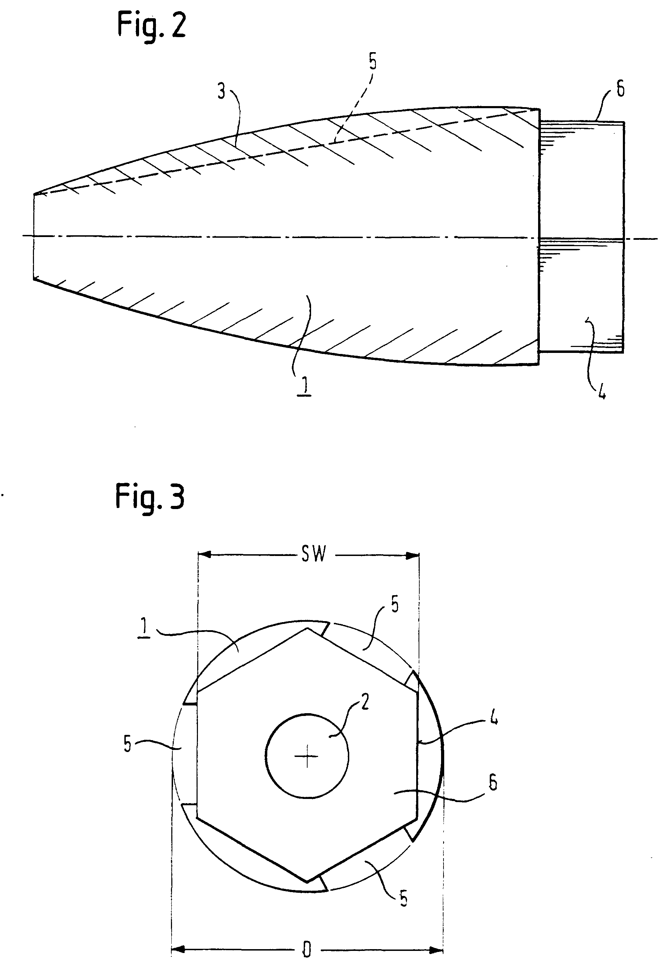

Das in den Fig. 1 bis 3 dargestellte Ausführungsbeispiel einer erfindungsgemäßen Interferenzschraube umfaßt einen Schraubenkörper 1 und einen Schraubenkopf 6, die einstückig ausgebildet sind und beispielsweise aus kompakten-femoralen Knochenmaterial, z.B. bovinen Ursprungs bestehen. Der Schraubenkörper 1 weist die Form eines Rotationsellipsoides auf und ist an seinem dem Schraubenkopf 6 entgegengesetzten Ende abgeflacht. In dem Schraubenkörper 1 und dem Schraubenkopf 6 ist eine Durchgangsbohrung 2 vorgesehen, die koaxial zur Mittelachse der Interferenzschraube verläuft und die sich durch die gesamte Interferenzschraube hindurch erstreckt.

Der Schraubenkopf 6 weist einen Außensechskant 4 auf und besitzt kleinere Querschnittsabmessungen als der breiteste Querschnitt des Schraubenkörpers 1.

Am Außenumfang des Schraubenkörpers 1 ist ein selbstschneidendes Außengewinde 3 vorgesehen, das sich über die gesamte Länge des Schraubenkörpers 1 erstreckt. Auf seiner gesamten Länge wird das Außengewinde 3 von insgesamt drei Spannuten 5 symmetrisch unterbrochen, die über den Umfang der Interferenzschraube gleichmäßig verteilt sind.

Grundsätzlich ist der Schraubenkörper 1 der Interferenzschraube in seinen Dimensionen an das Implantatlager angepaßt. Die Außenabmessungen einer solchen Interferenzschraube können je nach Einsatzort im femoralen oder tibialen Bohrkanal beispielsweise folgendermaßen sein:

| | |
|---|---|
| Länge (L) | 15 bis 30 mm, |
| Durchmesser (D) | 5 bis 12 mm, |
| Durchmesser der Durchgangsbohrung | 1 bis 3 mm, |
| Außensechskant | SW 5 bis 8. |

Das Außengewinde 3 kann selbstschneidend oder nicht selbstschneidend sein. Die Gewinde- bzw. Modulform ist bevorzugt ein Sägegewinde. Der Flankenwinkel beträgt bevorzugt 60°.

Die dargestellten Ausführungsbeispiele sind sowohl für die Implantation in den femoralen als auch in den tibialen Bohrkanal geeignet.

### Bezugszeichenliste

- 1: Schraubenkörper
- 2: Durchgangsbohrung
- 3: Außengewinde
- 4: Außensechskant
- 5: Spannuten
- 6: Schraubenkopf

## Patentansprüche

1. Interferenzschraube bestehend aus einem Schraubenkopf (6) und einem Schraubenkörper (1) mit einem Außengewinde (3), wobei in dem Schraubenkörper (1) eine Bohrung (2) für ein Führungsinstrument vorgesehen ist und das Außengewinde (3) von zumindest einer Spannut (5) unterbrochen ist,
**dadurch gekennzeichnet, daß**
Schraubenkopf (6) und Schraubenkörper (1) aus spongiösem, kortikalem oder kompakten Knochenmaterial bestehen,
der Schraubenkopf (6) einen Außenvierkant oder Außensechskant (4) aufweist, und
der Schraubenkopf (6) geringere Außenabmessungen als der Schraubenkörper (1) aufweist.

2. Interferenzschraube nach Anspruch 1,
**dadurch gekennzeichnet,**
**daß** der Schraubenkörper (1) im wesentlichen die Form eines Rotationsellipsoids aufweist.

3. Interferenzschraube nach Anspruch 1 oder 2,
**dadurch gekennzeichnet,**
**daß** der Schraubenkörper (1) ein selbstschneidendes Außengewinde (3) aufweist.

4. Interferenzschraube nach einem der vorstehenden Ansprüche,
**dadurch gekennzeichnet,**
**daß** sich die Spannut (5) über die gesamte axiale Länge des Außengewindes (3) erstreckt.

5. Interferenzschraube nach Anspruch 4,
**dadurch gekennzeichnet,**
**daß** der Querschnitt der Spannut (5) asymmetrisch, V-förmig oder rechteckig ist.

## Claims

1. An interference screw consisting of a screw head (6) and a screw body (1) comprising an external thread (3), with a bore (2) for a guiding instrument being provided in the screw body (1) and the external thread (3) being interrupted by at least one chip flute (5), **characterized in that**
the screw head (6) and the screw body (1) consist of spongious, cortical or compact bone material;
the screw head (6) has an external square or an external hexagon (4); and
the screw head (6) has smaller external dimensions than the screw body (1).

2. An interference screw in accordance with claim 1, **characterized in that** the screw body (1) has substantially the shape of a rotational ellipsoid.

3. An interference screw in accordance with claim 1 or claim 2, **characterized in that** the screw body (1) has a self-tapping external thread (3).

4. An interference screw in accordance with any one of the preceding claims, **characterized in that** the chip flute (5) extends over the total axial length of the external thread (3).

5. An interference screw in accordance with claim 4, **characterized in that** the cross-section of the chip flute (5) is asymmetrical, V-shaped or rectangular.

## Revendications

1. Vis d'interférence comprenant une tête de vis (6) et un corps de vis (1) avec un filetage extérieur (3), dans laquelle un perçage (2) est prévu pour un instrument de guidage dans le corps de vis (1) et le filetage extérieur (3) est interrompu par au moins une rainure de serrage (5),
**caractérisée en ce que**
la tête de vis (6) et le corps de vis (1) sont à base de matériau osseux spongieux, cortical ou compact, la tête de vis (6) présente un carré mâle ou un hexagone mâle (4), et
la tête de vis (6) présente des dimensions extérieures plus faibles que le corps de vis (1).

2. Vis d'interférence selon la revendication 1,
**caractérisée en ce que**
le corps de vis (1) présente sensiblement la forme d'un ellipsoïde de révolution.

3. Vis d'interférence selon la revendication 1 ou 2, **caractérisée en ce que**
le corps de vis (1) est un filetage extérieur (3) autotaraudeur.

4. Vis d'interférence selon l'une quelconque des revendications précédentes,
**caractérisée en ce que**
la rainure de serrage (5) s'étend sur toute la
longueur axiale du filetage extérieur (3).

5. Vis d'interférence selon la revendication 4,
**caractérisée en ce que**
la section de la rainure de serrage (5) est asymétrique, en forme de V ou rectangulaire.
